# EUROPEAN PATENT APPLICATION

(11) **EP 4 129 216 A1**
(43) Date of publication of application: **08.02.2023**
(21) Application number: 20926396.1
(22) Date of filing: 23.03.2020
(51) Int. Cl.: A61B 17/32

(54) **INSTRUMENT COMPRISING A NEEDLE FOR ULTRASOUND-GUIDED SURGERY PROVIDED WITH RETRACTABLE SCALPEL**

(71) Applicant: Fernández Gibello Alejandro, 10001 Cáceres (ES)
(72) Inventor: Fernández Gibello Alejandro, 10001 Cáceres (ES)
(74) Representative: Díaz de Bustamante y Terminel, Isidro
(86) International application number: PCT/ES2020/070201
(87) International publication number: WO 2021/191476

(57) **Abstract**

INSTRUMENT COMPRISING A NEEDLE FOR ULTRASOUND-GUIDED SURGERY PROVIDED WITH A RETRACTABLE SCALPEL with an internally hollow surgical needle (2), of 14G gauge or smaller, and a bevelled tip (2a)-scalpel blade (3) that can be incorporated into the needle (2) and retractable actuation means (4, 5, 6) of the scalpel (3) inside the needle (2), in which the scalpel blade (3) is provided with a hook (3a) at its distal end, and the retractable actuation means (4, 5, 6) move the scalpel (3) in the needle (2) with a 90° turning and linear sliding movement thereof between a retracted position, where the entire scalpel blade (3), including the hook (3a), is housed in the needle (2), and an extended position, where the hook (3a) and a portion of the scalpel blade (3) protrude through the bevelled tip (2a) of the needle (2).

## Description

### OBJECT OF THE INVENTION

The invention, as exposed on the wording of the present specification, refers to a needle instrument for ultrasound-guided surgery provided with a retractable scalpel which imparts to the function it is intended to, advantages and characteristics, which are described in detail below, which are an improvement of the current state of the art.

More specifically, the object of the invention focuses on a surgical instrument, in particular a cutting instrument applicable for ultrasound-guided surgery which, being essentially formed from a 14G gauge needle or other gauge inside which it incorporates a retractable scalpel blade and provided at its distal end with a hook, or the shape that is required, it is distinguished by comprising a sheath in which, in turn, the scalpel is incorporated inside the needle, there being a handling mechanism that, linked to said sheath, allows the extraction/retraction of said scalpel with respect to the needle, imparting greater safety both in the handling of the set as well as greater speed of use and control of the cutting instrument, in addition to drastically reducing the number of instruments that must be inserted in the patient, thus reducing the risks related to each gesture and reducing the time of surgery.

### APPLICATION FIELD OF THE INVENTION

The application field of the present invention is framed within the sector of the industry dedicated to the manufacture of medical instruments, focusing particularly on the field of cutting instruments intended for surgery, and more specifically cutting instruments intended for ultrasound-guided surgery.

### BACKGROUND OF THE INVENTION

As it is known, ultrasound-guided surgery is a technique whose purpose is to ensure that it is minimally invasive and that consists of operating without opening the skin, for which the surgeon sees what he is doing through an ultrasound machine. For this reason, cutting instruments, such as the scalpel, are inserted sheathed in a needle that can have different gauges and that, in some cases, is retractable. However, although said needles comply to a greater or lesser extent with the objective they pursue, that is, to penetrate under the skin without damaging the tissue with the cutting instrument until reaching the precise spot where it has to be used, they present aspects that are susceptible to be improved, in particular those related to the retraction system that allows controlling the use of the cutting instrument once the needle has been inserted.

The objective of the present invention is, thus, the development of an instrument of this type, specifically a 14G or different gauge needle, inside which it incorporates a scalpel blade provided with a retractable hook, which presents a series of improvements intended to provide an alternative solution, more effective and practical than those presented by the systems known so far, at least, in terms of the retraction control system of the cutting instrument and speed of use, which is essentially achieved thanks to the fact that the needle inside which the cutting instrument is incorporated, specifically a scalpel blade with a hook tip, in turn, houses inside a sheath that covers the scalpel, making it removable and retractable as it is linked to the needle handle, imparting greater safety, speed and control of use, as well as greater simplicity of manufacture, which in turn ensures better operation avoiding eventual actuation problems.

In reference to the state of the art, the following documents can be mentioned as the closest documents that disclose needles of this type:
- Document US2003074014A1, which refers to an articulable and reciprocal surgical knife applicable for endoscopic surgery that is incorporated inside a retractable cover, with the difference, apart from the fact that the scalpel is a blade with a cutting tip that does not have a hook and the cover is not specifically a needle, that the extraction and retraction of said tip is carried out through a system of control cables that are handled through a handle to which they are connected and, in any case, does not contemplate an additional internal sheath in which the scalpel is incorporated inside the needle, allowing its extraction and retraction, in addition to not allowing this surgical needle to be connected to a syringe and being able to introduce any medication or perform hydrodissections.
- Document US8603124B1, which describes a scalpel for ultrasound-guided surgery with a 14G or less gauge that ends in the shape of a hook and has a polyurethane sheath, but which, unlike the needle of the invention, is not retractable, and, in any case, it does not contemplate an additional internal sheath in which the scalpel is incorporated inside the needle, allowing its extraction and retraction.

- Document US6503262B1, which describes a retractable microsurgical tool comprising a shell with a removable inner blade through a sliding insert with a spring and button mechanism, differing from the needle of the invention, in addition to not consisting of a needle in the strict sense, the blade does not have a hook shape, and the gauge it presents is not specified, in that the extraction and retraction mechanism is more complex and, in any case, it does not contemplate an additional internal sheath in which the scalpel is incorporated inside the needle, allowing its extraction and retraction.
- And document WO2017176800A1, which discloses a subcutaneous cutting device comprising a retractable scalpel housed inside a cover, which, although it contemplates the shape of a hook for the scalpel, does not have a retractable cover, but rather the scalpel is enclosed in the needle itself, in addition, it does not contemplate an additional internal sheath in which the scalpel is incorporated inside the needle, allowing its extraction and retraction.

In view of the documents found, then, it is not observed that any of the inventions that they disclose, taken separately or in combination, describe the surgical needle of the present invention, as claimed.

### EXPLANATION OF THE INVENTION

The needle instrument for ultrasound-guided surgery provided with a retractable scalpel that the invention proposes allows the aforementioned objectives to be satisfactorily achieved, the characterizing details that make it possible and that conveniently distinguish it being collected in the final claims that accompany this description.

What the invention proposes, as noted above, is a surgical instrument, in particular a cutting instrument applicable for ultrasound-guided surgery of the type formed from a 14G or different gauge needle, inside which incorporates a scalpel blade provided at its distal end with a hook, which presents a controlled sliding movement with respect to the needle that allows its extraction and retraction, in order to be able to insert the needle with said scalpel in a retracted position and, once the intended position is reached, extract it to perform the cutting of the tissues. It also allows, thanks to this surgical needle adaptable to a syringe, the professional to introduce the needle while performing a hydrodissection of the tissue, that is, it separates with pressurized saline to position the instruments in a safe spot for subsequent surgical action. In this way, it is not necessary to insert 3 or 4 surgical instruments, but rather the surgical procedure can be performed with a single instrument that only creates a small perforation, thus avoiding the use of stitches and minimizing the risk of bleeding as well as infections.

And, based on this already known configuration, the instrument of the invention is essentially distinguished by the fact that, in order to provide said displacement movement of the scalpel with respect to the needle and provide it with a retractable character, it comprises an internal sheath in which the scalpel is sheathed to be housed inside the needle, such that it acts as a cover, there being a guide mechanism integrated in two cylinders, one integral with the sheath and the other connectable to the needle at their respective proximal ends, which fit coaxially with each other and can be handled by the operator to rotate and displace one with respect to the other and thereby ensure the sliding movement of the scalpel with respect to the needle between respective positions of use and determine its extraction or retraction by the distal end.

Thanks to this, the scalpel can be inserted safely with the needle and, when necessary, upon reaching the structure or tissue to be cut, everything being observed through the ultrasound machine, the needle is retracted and, consequently, the scalpel is extracted with the hookedtip, with which the precise cut will be made and, once the cutting operation is finished, the hook is covered again with the needle and the latter is extracted from under the patient's skin. In this way, an ultrasound-guided surgery can be performed more safely and much faster.

### DESCRIPTION OF THE DRAWINGS

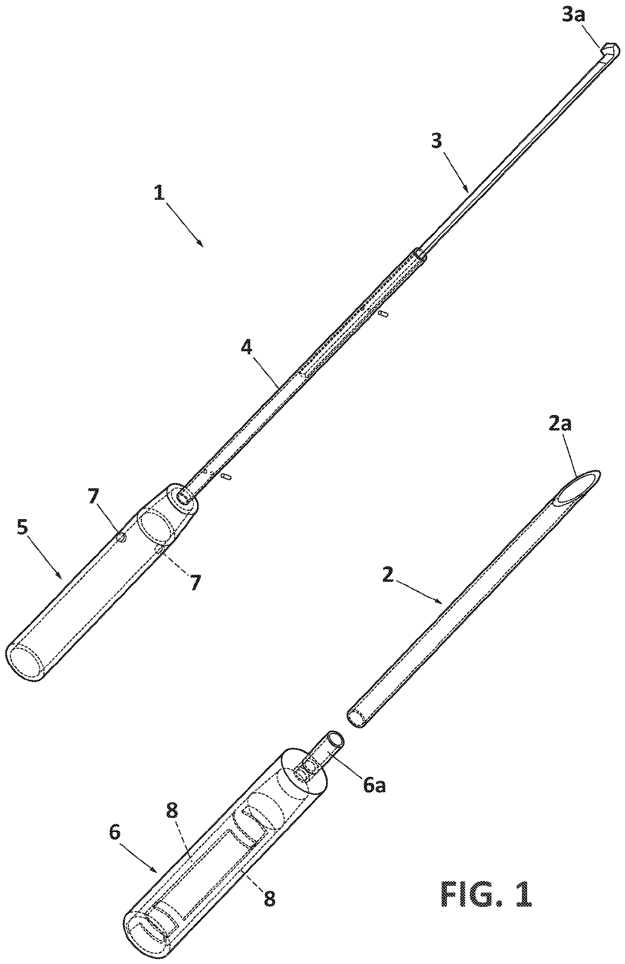

In order to complement the description being made and to ease a better understanding of the characteristics of the invention, is attached to the present specification, making part of the same, a set of layouts where, with an illustrative non limitative character, the following has been represented:
Figure number 1.- Shows an exploded perspective view of an embodiment of the needle instrument for ultrasound-guided surgery provided with a retractable scalpel, object of the invention, showing the parts and elements represented separately.
And figures number 2 and 3.- Show two perspective views of the instrument of the invention, according to the example shown in figure 1, in this case represented once assembled and in two positions of use, specifically with the scalpel extracted out of the needle in Figure 2 and with the scalpel and sheath retracted into the needle in Figure 3.

### PREFERRED EMBODIMENT OF THE INVENTION

In view of the aforementioned figures, and according to the numbering taken on them, it can be seen in them an example of non-limiting embodiment of the needle instrument for ultrasound-guided surgery provided with a retractable scalpel of the invention, which includes what is indicated and described in detail below.

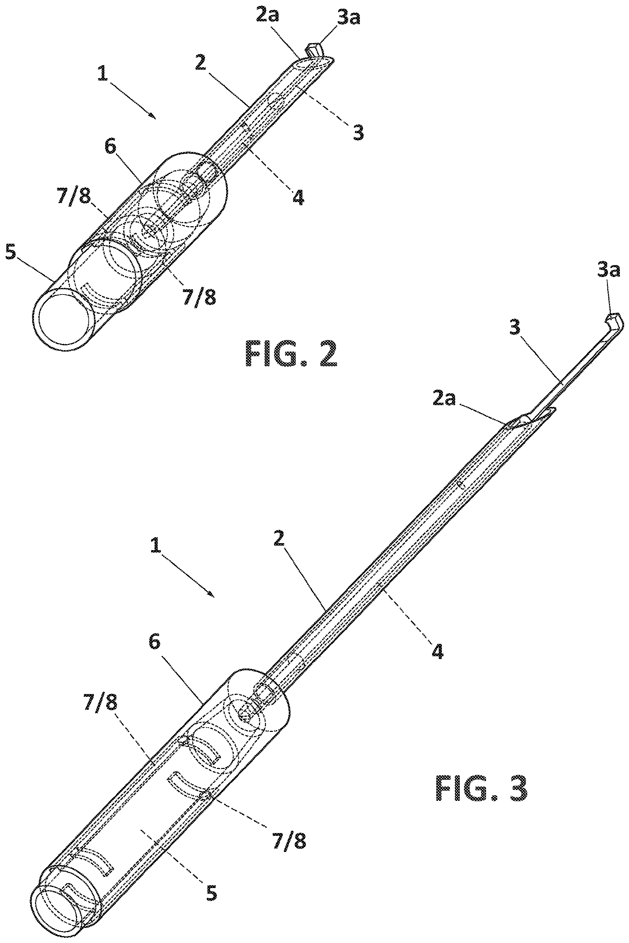

Thus, as can be seen in said figures, the instrument (1) in question essentially comprises:
- an internally hollow surgical needle (2) of 14G or a different gauge, and with a bevelled tip (2a);
- a scalpel blade (3), provided with a hook (3a) at its distal end, suitable for incorporation inside the needle (2);
- and retractable actuation means (4, 5, 6) which, by means of a simple 90° turning and linear sliding movement thereof, determine, on the longitudinal axis of the instrument (1), the displacement of the scalpel (3) inside the needle (2) between:
   - a retracted position, where the entire scalpel blade (3), including the hook (3a) at the end, is housed in said interior of the needle (2), allowing the penetration of the instrument in complete safety (figure 2),
   - and an extended position, where the hook (3a) and a portion of the scalpel blade (3) protrude through the bevelled tip (2a) of the needle (2), allowing the convenient cutting operation to be carried out (figure 3).

For their part, said retractable movement means (4, 5, 6) comprise at least:
- an internal sheath (4), in which the scalpel (3) is inserted and fixed, partially protruding at its end with the hook (3a),
- and two cylinders (5, 6) that are attached, respectively, to the proximal ends of the sheath (4) and the needle (2), which fit coaxially one (5) inside the other (6) there being between both a system of guides (7, 8) that controls and limits the turning and sliding movement of one with respect to the other to ensure the sliding movement of the scalpel (3) with respect to the needle (2) between its two positions of use.

Preferably, of the aforementioned cylinders, a first internal cylinder (5) is integrally attached to the proximal end of the sheath (4) in which the scalpel (3) is inserted, and a second external cylinder (6) is connectable to the proximal end of the needle (2) through a cannula (6a) provided for this purpose, which is sized to externally receive the cylindrical body of said needle (2) and internally be traversed by the sheath (4).

In any case, as can be seen in figure 2 and, especially in figure 3, the aforementioned system of guides (7, 8) that links both cylinders (5, 6) to each other to carry out the sliding movement of the scalpel (3) with respect to the needle (2), is determined by two protuberances (7) provided at diametrically opposite points on the external surface of the first cylinder (5), to which the sheath (4) is attached, and two channels (8) provided on the internal surface of the second cylinder (6), to which the needle (2) is connected, which describe two longitudinal sections, on opposite sides of said surface, and two transverse sections at both ends of said longitudinal sections, such that, with an initial turning movement in one direction, the aforementioned protuberances (7) run in the transverse sections of the channels (8) at one end, with a subsequent linear displacement movement, the protuberances (7) run along longitudinal sections of said channels (8), and in a final turning movement in the opposite direction, the protuberances (7) run in the transverse sections of the channels (8) at the opposite end.

Having sufficiently described the nature of the present invention, as well as a way of putting it into practice, it is not considered necessary to make a more extensive explanation in order that any person skilled in the art will understand its scope and the advantages derived from it, making known that, within reason it could be put into practice in other embodiments differing in detail from that indicated by way of example, and which will obtain the degree of protection that is sought, provided that its fundamental principle is not altered, changed or modified.

## Claims

1. INSTRUMENT COMPRISING A NEEDLE FOR ULTRASOUND-GUIDED SURGERY PROVIDED WITH A RETRACTABLE SCALPEL that, comprising an internally hollow surgical needle (2) of 14G or a different gauge, and with a bevelled tip (2a); a scalpel blade (3) suitable for incorporation inside the needle (2); and retractable actuation means (4, 5, 6) that determine, on the longitudinal axis of the instrument (1), the displacement of the scalpel (3) inside the needle (2), is **characterized in that** the scalpel blade (3) is provided with a hook (3a) at its distal end, and **in that** the retractable actuation means (4, 5, 6) determine the displacement of the scalpel (3) inside the needle (2) by means of a 90° turning and linear sliding movement thereof, allowing said displacement between a retracted position, where the entire scalpel blade (3), including the hook (3a) at the end, is housed in the interior of the needle (2), and an extended position, where the hook (3a) and a portion of the scalpel blade (3) protrude through the bevelled tip (2a) of the needle (2).

2. INSTRUMENT COMPRISING A NEEDLE FOR ULTRASOUND-GUIDED SURGERY PROVIDED WITH A RETRACTABLE SCALPEL, according to claim 1, **characterized in that** said retractable movement means (4, 5, 6) comprise at least one internal sheath (4), in which it is inserted and fixed the scalpel (3) protruding partially at its end with the hook (3a), and two cylinders (5, 6) that are attached, respectively, to the proximal ends of the sheath (4) and of the needle (2), which fit coaxially one (5) inside the other (6) there being between both a system of guides (7, 8) that controls and limits the turning and sliding movement of one with respect to the other to ensure the sliding movement of the scalpel (3) with respect to the needle (2) between its two positions of use.

3. INSTRUMENT COMPRISING A NEEDLE FOR ULTRASOUND-GUIDED SURGERY PROVIDED WITH A RETRACTABLE SCALPEL, according to claim 2, **characterized in that** a first internal cylinder (5) is attached integrally to the proximal end of the sheath (4) in which the scalpel (3) is inserted, and a second external cylinder (6) is connectable to the proximal end of the needle (2) through a cannula (6a) provided for this purpose, which is dimensioned to externally receive the cylindrical body of said needle (2) and internally be traversed by the sheath (4).

4. INSTRUMENT COMPRISING A NEEDLE FOR ULTRASOUND-GUIDED SURGERY PROVIDED WITH A RETRACTABLE SCALPEL, according to claim 2 or 3, **characterized in that** the system of guides (7, 8) that links both cylinders (5, 6) to each other to carry out the sliding movement of the scalpel (3) with respect to the needle (2) is determined by two protuberances (7) provided on the external surface of the first cylinder (5), to which the sheath (4) is attached, and two channels (8) provided on the internal surface of the second cylinder (6), to which the needle (2) is connected, so that when the movement is carried out, the aforementioned protuberances (7) run in said channels (8).
